# EUROPEAN PATENT SPECIFICATION

(11) **EP 3 473 165 B1**
(45) Date of publication and mention of the grant of the patent: **18.11.2020**
(21) Application number: 17813516.6
(22) Date of filing: 08.06.2017
(51) Int. Cl.: A61B 5/00, H04M 1/725, A61B 5/107

(54) **SKINFOLD CALIPER**
HAUTFALTENMESSSCHIEBER
CALIBRE DE MESURE DE PLI CUTANÉ

(30) Priority: 16.06.2016 KR 20160074927
(43) Date of publication of application: 24.04.2019
(73) Proprietor: Jeong, Han Nah, Seoul 03970 (KR)
(72) Inventor: Jeong, Han Nah, Seoul 03970 (KR)
(74) Representative: M. Zardi & Co S.A.
(86) International application number: PCT/KR2017/005945
(87) International publication number: WO 2017/217691

(56) References cited:
- CN-Y- 201 216 594
- JP-A- 2012 148 098
- US-A- 4 315 372
- US-A- 5 430 954
- US-A1- 2013 123 668

## Description

### BACKGROUND

Embodiments of the inventive concept disclosed herein relate to a skinfold caliper, and more particularly, relate to a skinfold caliper for measuring a skin thickness of a to-be-measured portion of a user.

Accurate body fat measurement is the first step in promoting health. However, a conventional body fat measuring device may not guarantee accuracy.

However, a body fat caliper, which measures a thickness of subcutaneous fat, has an advantage of ensuring an accuracy of about 99 % of a result, but it has not been popularized due to a complicated calculation process.

A skinfold caliper is being widely used as such body fat measuring device.

The skinfold caliper is the most accurate way (skinfold thickness method) to measure the thickness of the subcutaneous fat by an individual.

This skinfold caliper has a pair of holders that approach to each other or move away from each other. Each of the holders has a tip that contacts or moves away to/from the to-be-measured object.

However, the conventional skinfold caliper is operated in an analog manner. That is, after measuring a skin thickness of a to-be-measured portion of a user, the measured numerical value is put into a complicated formula so that a result related to a body composition is derived.

This complexity in use makes it less usable, regardless of a simplicity and accuracy of the measurement. Thus, although the skinfold caliper has the highest accuracy to be adopted as an international standard anthropometric measuring device compared to the conventional body fat measuring devices, and has the greatest merit of being easy to measure, due to the complicated mathematical computation procedure, only experts use it.

In addition, the conventional skinfold caliper has a problem that the accuracy of the measurement is lowered depending on a measurer.

That is, the conventional skinfold caliper has a problem that measurement data of the skin thickness varies depending on a degree of a force applied by the measurer to the skin after positioning the skin of the to-be-measured portion of a subject between the tips of the pair of holders. In some cases, after the use of the skinfold caliper, a mark of each of tips of the holders may remain on the measured body part of the subject due to the force applied by the measurer. US2013/123668 A1 shows an example of a conventional skinfold caliper.

Therefore, since the conventional skinfold caliper requires expertise in measuring, not only a general user may not use it easily, but also the accuracy of measurement data is low.

### SUMMARY

In order to solve the above-mentioned problem, embodiments of the inventive concept provide a skinfold caliper which may be easily used by a non-expert, and may precisely measure a thickness of a to-be-measured object.

According to an aspect of an embodiment, a skinfold caliper includes a first holder and a second holder respectively having tips approaching or moving away to or from a to-be-measured portion of a to-be-measured object, wherein the first holder and the second holder pivot relative to each other about a pivot axis, a scale unit provided in the first holder, wherein the scale unit has a plurality of angular scales formed along a rotation path of the second holder, a probe unit disposed in the second holder so as to electrically connect to the scale unit, wherein the probe unit senses a relative rotation angle of the second holder relative to the first holder, a pressure measuring sensor provided at the tip of one of the first holder and the second holder, wherein the pressure measuring sensor measures a pressure applied to the to-be-measured portion of the to-be-measured object, and a controller configured for, when the pressure measured by the pressure measuring sensor reaches a predetermined pressure, receiving rotation angle measurement data corresponding to an angular scale of the scale unit from the probe unit, and then calculating the rotation angle measurement data into a linear distance between the tips of the first holder and the second holder.

According to another aspect of an embodiment, the skinfold caliper includes an elastic member interconnecting the first holder and the second holder, wherein the elastic member provides an elastic force such that the first holder and the second holder are spaced apart from each other.

According to another aspect of an embodiment, the first holder is composed of a pair of separable cases, and the second holder is disposed between the pair of separable cases so as to pivot about the pivot axis.

According to another aspect of an embodiment, the probe unit includes first probes contacting a plurality of first angular scales equidistantly arranged at a first angular unit interval, and second probes contacting a plurality of second angular scales equidistantly arranged at a second angular unit interval, wherein the first angular unit is equal to a multiple of the second angular unit by a number of the plurality of second angular scales.

According to another aspect of an embodiment, the skinfold caliper further includes a display provided on the first holder or the second holder for displaying the linear distance data between the tips of the first holder and the second holder calculated by the controller.

According to another aspect of an embodiment the skinfold caliper further includes a communication module provided in the first holder or the second holder, wherein the communication module transmits, to an external device, the linear distance data between the tips of the first holder and the second holder calculated by the controller or body composition information calculated automatically based on the linear distance data.

According to another aspect of an embodiment, the external device displays the linear distance data or the body composition information calculated automatically based on the linear distance data.

According to another aspect of an embodiment, the skinfold caliper further includes a battery for providing electricity to the scale unit, the probe unit, the pressure measuring sensor, and the controller.

The skinfold caliper according to the inventive concept may be easily used by the non-expert, and may precisely measure the thickness of the to-be-measured object.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIG. 1 is a perspective view of a skinfold caliper according to an embodiment of the inventive concept,
FIG. 2 is an exploded perspective view of FIG. 1,
FIG. 3 is a perspective view in which a part of a case of FIG. 1 is removed,
FIG. 4 shows a perspective view in which a first holder and a second holder of FIG. 1 are adjacent to each other,
FIG. 5 shows a perspective view in which a part of a case of FIG. 3 is removed,
FIG. 6 shows a diagram in which a to-be-measured object is measured using a skinfold caliper according to an embodiment of the inventive concept,
FIG. 7 is a diagram illustrating a communication between an external device and a skinfold caliper according to an embodiment of the inventive concept, and
FIG. 8 is a diagram illustrating a screen displayed on an external device.

### DETAILED DESCRIPTION

Hereinafter, the inventive concept will be described in detail with reference to the accompanying drawings.

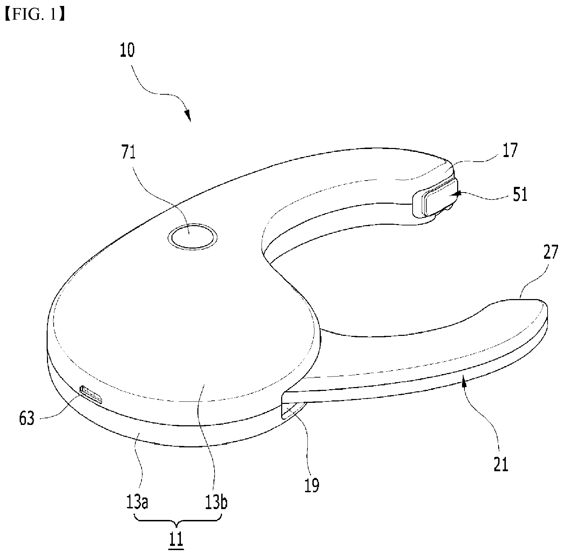

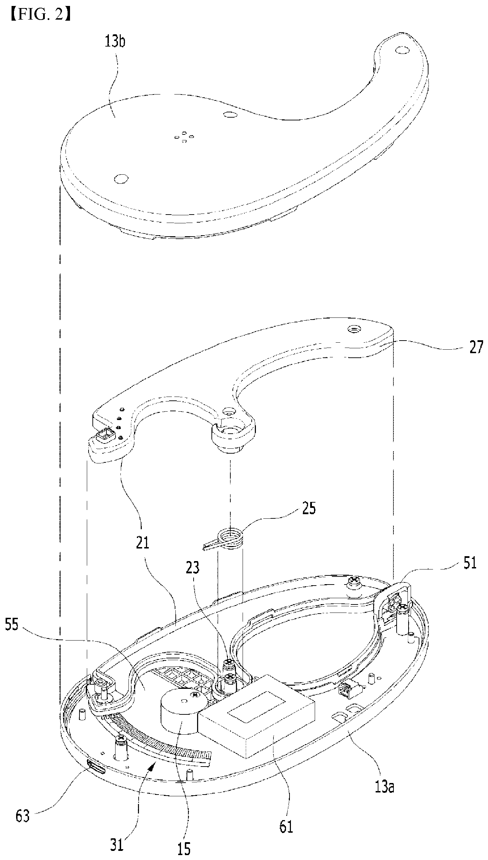

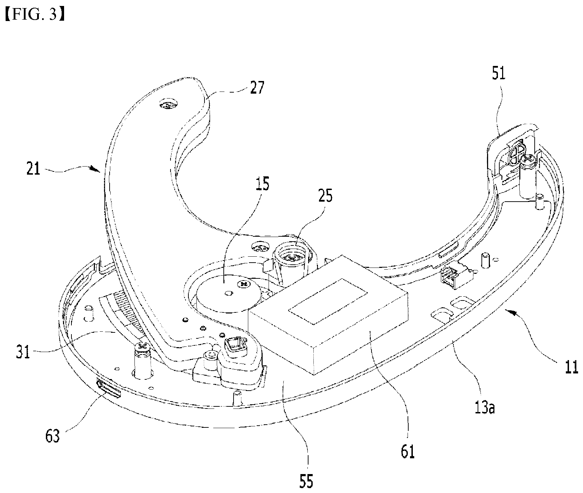

FIG. 1 to FIG. 3 illustrate a skinfold caliper according to an embodiment of the inventive concept.

As shown in these figures, a skinfold caliper 10 according to an embodiment of the inventive concept includes a first holder 11, a second holder 21, a scale unit 31, a probe unit 41, a pressure measuring sensor 51, and a controller 55.

The first holder 11 has a cylindrical shape having a receiving space defined therein, and the first holder 11 includes a pair of separable cases 13a and 13b. The pair of cases 13a and 13b are coupled to each other by a fixing pin 15. The first holder 11 has a streamlined cross-sectional shape so as to be gripped. In addition, a first tip 17 that approaches or moves away to or from a to-be-measured object 1 is provided at an end of the first holder 11. In addition, one side edge of the first holder 11 facing the second holder 21 is partially recessed so that the to-be-measured object 1 does not interfere with the first holder 11. In addition, an opening 19 having a predetermined length is defined at the other side edge of the first holder 11 so as to prevent interference with the first holder 11 when the second holder 21 pivots.

The second holder 21 has a plate shape having a constant thickness, and is provided between the pair of cases 13a and 13b of the first holder 11 so as to pivot about a pivot axis 23. A second tip 27 that approaches or moves away to or from the first tip 17 is provided at an end of the second holder 21. In addition, one side edge of the second holder 21 facing the first holder 11 is partially recessed so that the to-be-measured object 1 does not interfere with the second holder 21. As a result, respective recessed portions of the first holder 11 and the second holder 21 define a hole having an elliptical sectional shape when the first holder 11 and the second holder 21 approach to each other.

The pivot axis 23 is provided with an elastic member 25 which interconnects the case 13a of one side of the first holder 11 and the second holder 21, and provides an elastic force such that the first holder 11 and the second holder 21 are spaced apart from each other. In this embodiment, a torsion spring is disclosed as the elastic member 25, but the elastic member 25 is not limited thereto. The elastic member 25 may be applied as various types of springs.

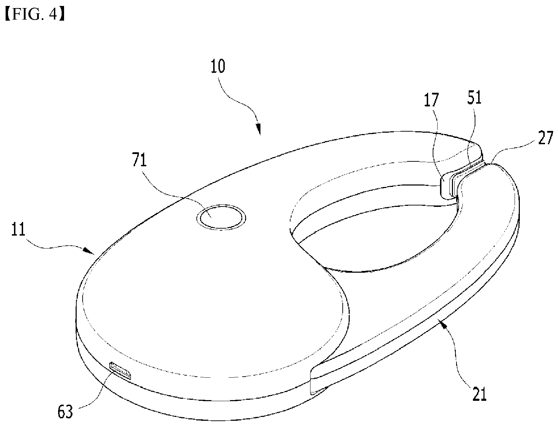

Thus, the skinfold caliper 10 according to an embodiment of the inventive concept has a tongs shape as a whole. In addition, as shown in FIG. 1, when an external force is not applied to the first holder 11 and the second holder 21, respective tips 17 and 27 of the first holder 11 and the second holder 21 are spaced apart from each other by the elastic member 25. Further, as shown in FIG. 4, when the external force exceeding an elastic force is applied to the first holder 11 and the second holder 21, respective tips 17 and 27 of the first holder 11 and the second holder 21 approach to each other.

The scale unit 31 has a strip shape having a circular arc section, and is provided in the first holder 11. The scale unit 31 is made of electrically conductive material, and is electrically connected to the controller 55. The scale unit 31 has a plurality of angular scales 33 and 35 formed along a rotation path of the second holder 21. That is, the scale unit 31 has a plurality of first angular scales 33 equidistantly arranged at a first angular unit interval, and, at the same time, has a plurality of second angular scales 35 equidistantly arranged at a second angular unit interval, wherein the first angular unit corresponds to a multiple of the second angular unit by a number of the plurality of second angular scales 35. The plurality of angular scales 33 of the first angular unit and the plurality of angular scales 35 of the second angular unit are formed in parallel along a longitudinal direction of the scale unit 31. In this connection, as an example, the angular scales 33 may be arranged at an angular unit interval of 1 degree, and the angular scales 35 may be arranged at an angular unit interval of 0.1 degree. However, the angular scales 33 equidistantly arranged at a first angular unit interval and the angular scales 35 equidistantly arranged at a second angular unit interval are not limited thereto, and may have various sizes.

The probe unit 41 is provided in the second holder 21 so as to electrically connect to the scale unit 31, and senses a relative rotation angle of the second holder 21 relative to the first holder 11. The probe unit 41 is provided at an end opposite to the second tip 27 with the pivot axis 23 therebetween. The probe unit 41 is electrically connected to the scale unit 31 provided in the first holder 11 as the second holder 21 pivots relative to the first holder 11, and measures a separation angle between the first holder 11 and the second holder 21.

The probe unit 41 includes first probes 43 contacting the plurality of angular scales 33 of the first angular unit of the scale unit 31 and second probes 45 contacting the plurality of angular scales 35 of the second angular unit of the scale unit 31. The probe unit 41 is electrically connected to the scale unit 31. In addition, the scale unit 31 may be configured such that a current value, a resistance value, or the like of the scale unit 31 corresponds to each of the angular scales 33 of the first angular unit and the angular scales 35 of the second angular unit. Thus, the current value or the resistance value corresponding to each of the corresponding angular scales 33 and 35 of the scale unit 31 in contact with the probe unit is measured. In this way, the rotation angle measurement data, for example, the separation angle between the first tip 17 of the first holder 11 and the second tip 27 of the second holder 21 may be accurately measured based on the measurement of the current value or the resistance value and depending on a rotation angle of the probe unit 41.

The pressure measuring sensor 51 is provided at the front of the first tip 17 of the first holder 11 to measure a pressure applied to the to-be-measured portion of the to-be-measured object 1 together with the second tip 27 of the second holder 21. In this embodiment, the pressure measuring sensor 51 is shown as being provided at the front of the first tip 17 of the first holder 11, but the position of the pressure measuring sensor 51 is not limited thereto. In addition, the pressure measuring sensor 51 may be provided at the front of the second tip 27 of the second holder 21.

The pressure measuring sensor 51 is electrically connected to the controller 55, and transmits pressure data measured by the pressure measuring sensor 51 to the controller 55.

The controller 55 is provided on a printed circuit board, and, at the same time, is accommodated in the first holder 11. The controller 55 is electrically connected to the scale unit 31, the probe unit 41, and the pressure measuring sensor 51. When the pressure measured by the pressure measuring sensor 51 reaches a predetermined pressure, in order to measure a thickness of a to-be-measured portion of the to-be-measured object 1, the controller 55 receives the rotation angle measurement data corresponding to the angular scales 33 and 35 of the scale unit 31 from the probe unit 41, and then calculates the rotation angle measurement data into a linear distance between the first tip 17 of the first holder 11 and the second tip 27 of the second holder 21. Thus, the thickness of the to-be-measured portion of the to-be-measured object 1 between the first tip 17 of the first holder 11 and the second tip 27 of the second holder 21 may be measured.

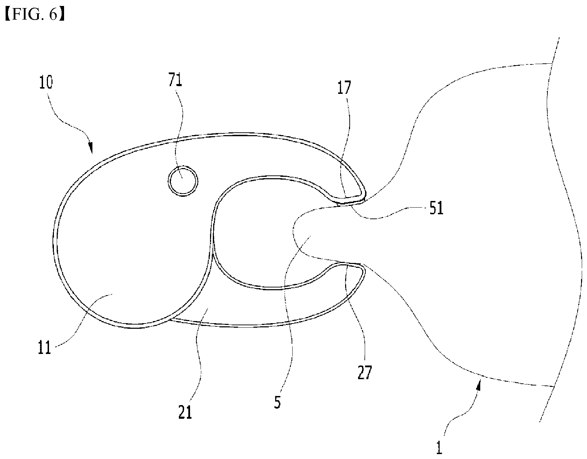

In this connection, although not shown, the controller 55 may be electrically connected to a buzzer or a lamp. Thus, when the pressure measured by the pressure measuring sensor 51 is equal to or higher than a set pressure, the controller 55 emits sound or light through the buzzer or the lamp to warn the measurer not to press a to-be-measured portion 5 (with reference to FIG. 6) of the to-be-measured object 1 (with reference to FIG. 6) any longer.

In addition, a battery 61 for supplying electricity to the scale unit 31, the probe unit 41, the pressure measuring sensor 51, and the controller 55 is accommodated in the first holder 11. The battery 61 is arranged adjacent to the fixing pin 15 so as not to be located in the rotation path range of the second holder 21. The battery 61 may be charged through a charging port 63 provided in the first holder 11.

In one example, although not shown, the skinfold caliper 10 according to an embodiment of the inventive concept may be provided with a display on the first holder 11 or on the second holder 21 to display the linear distance data between the tips 17 and 27 of the first holder 11 and the second holder 21 calculated by the controller 55. Thus, the thickness of the to-be-measured portion 5 of the to-be-measured object 1 may be confirmed through the display in the field.

In addition, a communication module may be provided in the first holder 11 or the second holder 21 to transmit the linear distance data between the first tip 17 of the first holder 11 and the second tip 27 of the second holder 21 calculated by the controller 55 to an external device. Thus, the thickness of the to-be-measured portion of the subject may be confirmed through the external device in real time.

In this connection, reference numeral 71, which is not described, denotes an operation button for controlling supply of power from the battery 61 to the scale unit 31, the probe unit 41, the pressure measuring sensor 51, and the controller 55.

With this configuration, a process of measuring the to-be-measured portion of the to-be-measured object 1 using the skinfold caliper 10 according to an embodiment of the inventive concept will be described.

First, the operation button 71 is activated to supply the power to the skinfold caliper 10 according to an embodiment of the inventive concept, then, as shown in FIG. 1, in a state where the first holder 11 and the second holder 21 are spaced apart from each other, the first tip 17 of the first holder 11 and the second tip 27 of the second holder 21 are located opposite to each other with the to-be-measured portion 5 of the to-be-measured object 1 therebetween.

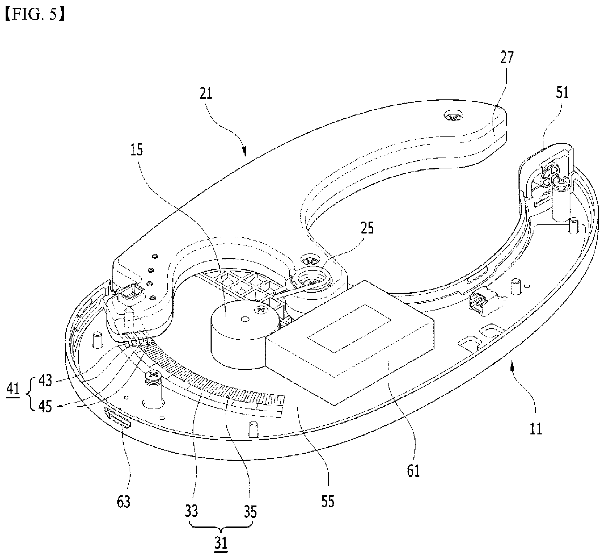

Then, the second holder 21 is pivoted relative to the first holder 11 by applying the external force as shown in FIG. 5 and FIG. 6 so that the first tip 17 of the first holder 11 and the second tip 27 of the second holder 21 are brought into close contact with the to-be-measured portion 5 of the to-be-measured object 1. At this time, as the second holder 21 is pivoted relative to the first holder 11, the probe unit 41 moves along the scale unit 31 as shown in FIG. 5.

When the first tip 17 of the first holder 11 and the second tip 27 of the second holder 21 are brought into close contact with the to-be-measured portion 5 of the to-be-measured object 1, and when the pressure measured by the pressure measuring sensor 51 provided at the first tip 17 of the first holder 11 reaches the set pressure, the controller 55 receives the rotation angle measurement data corresponding to each of the angular scales 33 and 35 of the scale unit 31 indicated by the probe unit 41 from the probe unit 41. Then, the controller 55 converts the rotation angle measurement data to the linear distance between the tips 17 and 27 of the first holder 11 and the second holder 21 to acquire the linear distance data, for example, the thickness of the to-be-measured portion 5. Thus, the thickness of the to-be-measured portion 5 of the to-be-measured object 1 may be precisely measured.

In one example, the thickness data of the to-be-measured portion 5 of the to-be-measured object 1 calculated by the controller 55 may be displayed through the display or displayed through the external device via the communication module.

As shown in FIG. 1, after the measurement of the thickness of the to-be-measured portion 5 of the to-be-measured object 1 is completed, when the external force is removed from the first holder 11 and the second holder 21, the first tip 17 of the first holder 11 and the second tip 27 of the second holder 21 are moved away from each other by the elastic force of the elastic member 25 to their original states.

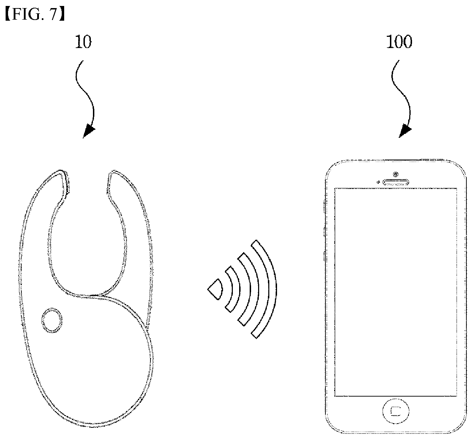

As shown in FIG. 7, the skinfold caliper 10 according to an embodiment of the inventive concept and an external device 100 may communicate with each other. The skinfold caliper 10 may transmit the linear distance data or the thickness data of the to-be-measured portion 5 of the to-be-measured object 1 to the external device 100 using any wireless communication method, such as Bluetooth, RF communication, IR communication, UWB (Ultra Wideband), ZigBee communication, and the like. Alternatively, the skinfold caliper 10 may automatically calculate body composition information such as a body fat rate, a muscle mass, and the like based on the thickness data of the to-be-measured portion 5, and transmit the calculated body composition information to the external device 100.

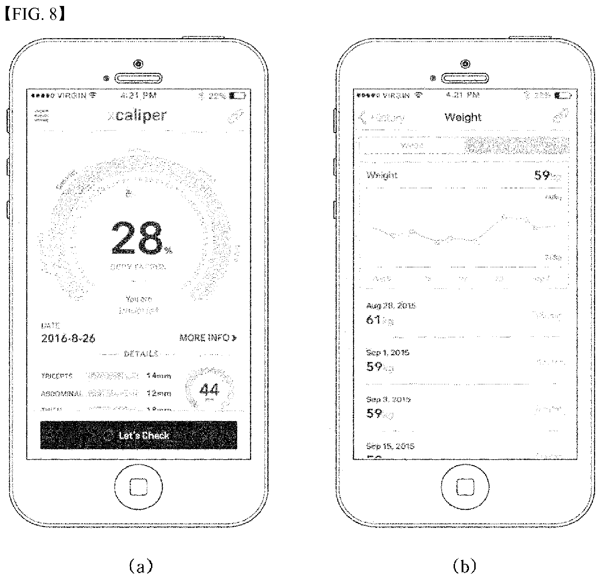

As shown in FIG. 8, the external device 100 may display various information such as the body composition information, an obesity degree, a measurement history, and the like on a screen. As an example, thickness data of the to-be-measured portion 5 (e.g., triceps, abdomen, femoral muscle, and the like), and body composition information automatically calculated based on the thickness data are displayed on a screen of the external device 100 in FIG. 8A. In addition, a weight change history based on a predetermined period (e.g., week or month) is displayed on the screen of the external device 100 in FIG. 8B.

A computer program (e.g., a smartphone application, and the like) may be provided for communicating with the skinfold caliper 10, and controlling overall operations of the external device 100.

The user may run the computer program and sequentially measures the thicknesses of the several to-be-measured portions 5 using the skinfold caliper 10, and then may confirm the measurement result through the screen of the external device 100.

Thus, the skinfold caliper 10 according to the inventive concept may be easily used by a non-expert, and may precisely measure the thickness of the to-be-measured portion 5 of the to-be-measured object 1.

Further, the pressure measuring sensor 51 may be used to ensure that the to-be-measured portion 5 is not pressed at or above a predetermined pressure. Thus, no mark of the tips 17 and 27 of the holders 11 and 21 remains on the to-be-measured portion 5. This improves a user's sensitivity.

The user may confirm the body composition information more easily and accurately through the display of the skinfold caliper 10 or external device 100 even though the user does not directly perform complicated calculations with the thickness data of the to-be-measured portion 5 of the to-be-measured object 1 as a factor in order to obtain the body composition information such as the body fat rate, the muscle mass, and the like.

While the inventive concept has been described with reference to embodiments, it will be apparent to those skilled in the art that various changes and modifications may be made without departing from the scope of the inventive concept. Therefore, it should be understood that the above embodiments are not limiting, but illustrative.

## Claims

1. A skinfold caliper (10) comprising:
a first holder (11) and a second holder (21) respectively having tips approaching or moving away to or from a to-be-measured portion of a to-be-measured object, wherein the first holder and the second holder pivot relative to each other about a pivot axis; (23)
a scale unit (31) provided in the first holder, wherein the scale unit has a plurality of angular scales formed along a rotation path of the second holder;
a probe unit (41) disposed in the second holder so as to electrically connect to the scale unit, wherein the probe unit senses a relative rotation angle of the second holder relative to the first holder;
a pressure measuring sensor (51) provided at the tip of one of the first holder and the second holder, wherein the pressure measuring sensor measures a pressure applied to the to-be-measured portion of the to-be-measured object; and
a controller (55) configured for:
when the pressure measured by the pressure measuring sensor reaches a predetermined pressure, receiving rotation angle measurement data corresponding to an angular scale of the scale unit from the probe unit; and
calculating the rotation angle measurement data into a linear distance between the tips of the first holder and the second holder.

2. The skinfold caliper of claim 1, further comprising an elastic member interconnecting the first holder and the second holder, wherein the elastic member provides an elastic force such that the first holder and the second holder are spaced apart from each other.

3. The skinfold caliper of claim 1, wherein the first holder is composed of a pair of separable cases,
wherein the second holder is disposed between the pair of the separable cases so as to pivot about the pivot axis.

4. The skinfold caliper of claim 1, wherein the probe unit includes:
first probes contacting a plurality of first angular scales equidistantly arranged at a first angular unit interval; and
second probes contacting a plurality of second angular scales equidistantly arranged at a second angular unit interval, wherein the first angular unit is equal to a multiple of the second angular unit by a number of the plurality of second angular scales.

5. The skinfold caliper of claim 1, further comprising a display provided on the first holder or the second holder for displaying the linear distance between the tips of the first holder and the second holder calculated by the controller.

6. The skinfold caliper of claim 1, further comprising a communication module provided in the first holder or the second holder, wherein the communication module transmits, to an external device, the linear distance data between the tips of the first holder and the second holder calculated by the controller or body composition information calculated automatically based on the linear distance data.

7. The skinfold caliper of claim 6, wherein the external device displays the linear distance data or the body composition information calculated automatically based on the linear distance data.

8. The skinfold caliper of claim 1, further comprising a battery for providing electricity to the scale unit, the probe unit, the pressure measuring sensor, and the controller.

## Patentansprüche

1. Hautfalten-Caliper (10) umfassend:
einen ersten Halter (11) und einen zweiten Halter (21), die jeweils Spitzen aufweisen, die sich annähern an oder sich wegbewegen von einem zu messenden Abschnitt eines zu messenden Objekts, wobei der erste Halter und der zweite Halter relativ zueinander um eine Schwenkachse (23) schwenken;
eine Skaleneinheit (31), die in dem ersten Halter vorgesehen ist, wobei die Skaleneinheit eine Vielzahl von Winkelskalen aufweist, die entlang eines Drehwegs des zweiten Halters gebildet sind;
eine Sondeneinheit (41), die in dem zweiten Halter angeordnet ist, um sich elektrisch mit der Skaleneinheit zu verbinden, wobei die Sondeneinheit einen relativen Drehwinkel des zweiten Halters relativ zu dem ersten Halter abfühlt;
einen Druckmesssensor (51), der an der Spitze von einem des ersten Halters und des zweiten Halters vorgesehen ist, wobei der Druckmesssensor einen Druck misst, der auf den zu messenden Abschnitt des zu messenden Objekts aufgebracht wird; und
eine Steuereinrichtung (55), die konfiguriert ist zum:
wenn der durch den Druckmesssensor gemessene Druck einen vorgegebenen Druck erreicht, Empfangen von einer Winkelskala der Skaleneinheit entsprechenden Drehwinkelmessdaten von der Sondeneinheit; und
Berechnen der Drehwinkelmessdaten in einen linearen Abstand zwischen den Spitzen des ersten Halters und des zweiten Halters.

2. Hautfalten-Caliper nach Anspruch 1, ferner umfassend ein elastisches Element, das den ersten Halter und den zweiten Halter miteinander verbindet, wobei das elastische Element eine elastische Kraft derart bereitstellt, dass der erste Halter und der zweite Halter voneinander beabstandet sind.

3. Hautfalten-Caliper nach Anspruch 1, wobei der erste Halter aus einem Paar von trennbaren Gehäusen zusammengesetzt ist,
wobei der zweite Halter zwischen dem Paar der trennbaren Gehäuse angeordnet ist, um um die Schwenkachse zu schwenken.

4. Hautfalten-Caliper nach Anspruch 1, wobei die Sondeneinheit umfasst:
erste Sonden, die eine Vielzahl von ersten Winkelskalen kontaktieren, die abstandsgleich in einem ersten Winkeleinheitsintervall angeordnet sind; und
zweite Sonden, die eine Vielzahl von zweiten Winkelskalen kontaktieren, die abstandsgleich in einem zweiten Winkeleinheitsintervall angeordnet sind, wobei die erste Winkeleinheit gleich einem Vielfachen der zweiten Winkeleinheit um eine Anzahl der Vielzahl der zweiten Winkelskalen ist.

5. Hautfalten-Caliper nach Anspruch 1, ferner umfassend eine Anzeige, die auf dem ersten Halter oder dem zweiten Halter vorgesehen ist, um den durch die Steuereinrichtung berechneten linearen Abstand zwischen den Spitzen des ersten Halters und des zweiten Halters anzuzeigen.

6. Hautfalten-Caliper nach Anspruch 1, ferner umfassend ein in dem ersten Halter oder dem zweiten Halter vorgesehenes Kommunikationsmodul, wobei das Kommunikationsmodul die durch die Steuereinrichtung berechneten linearen Abstandsdaten zwischen den Spitzen des ersten Halters und des zweiten Halters oder eine basierend auf den linearen Abstandsdaten automatisch berechnete Körperzusammensetzungsinformation an eine externe Vorrichtung überträgt.

7. Hautfalten-Caliper nach Anspruch 6, wobei die externe Vorrichtung die linearen Abstandsdaten oder die basierend auf den linearen Abstandsdaten automatisch berechnete Körperzusammensetzungsinformation anzeigt.

8. Hautfalten-Caliper nach Anspruch 1, ferner umfassend eine Batterie zum Bereitstellen von Elektrizität für die Skaleneinheit, die Sondeneinheit, den Druckmesssensor und die Steuereinrichtung.

## Revendications

1. Compas de mesure de pli cutané (10) comprenant :
une première branche (11) et une deuxième branche (21) ayant respectivement des extrémités s'approchant ou s'éloignant d'une partie à mesurer d'un objet à mesurer, où la première branche et la deuxième branche pivotent l'une par rapport à l'autre autour d'un axe de pivotement (23),
une unité d'échelle (31) prévue dans la première branche, où l'unité d'échelle présente une pluralité d'échelles angulaires formées le long d'une trajectoire de rotation de la deuxième branche,
une unité de sonde (41) disposée dans la deuxième branche de manière à se connecter électriquement à l'unité d'échelle, où l'unité de sonde détecte un angle relatif de rotation de la deuxième branche par rapport à la première branche,
un détecteur de mesure de la pression (51) prévu à l'extrémité de l'une des deux entre la première branche et la deuxième branche, où le détecteur de mesure de la pression mesure une pression appliquée à la partie à mesurer de l'objet à mesurer et
un contrôleur (55) configuré pour :
quand la pression mesurée par le détecteur de mesure de la pression atteint une pression prédéterminée, recevoir les données de mesure de l'angle de rotation correspondant à une échelle angulaire de l'unité d'échelle de l'unité de sonde et
calculer les données de mesure de l'angle de rotation en une distance linéaire entre les extrémités de la première branche et de la deuxième branche.

2. Compas de mesure de pli cutané selon la revendication 1, comprenant en outre un élément élastique interconnectant la première branche et la deuxième branche, où l'élément élastique fournit une force élastique telle que la première branche et la deuxième branche sont séparées l'une de l'autre.

3. Compas de mesure de pli cutané selon la revendication 1, **caractérisé en ce que** la première branche est composée d'une paire d'étuis séparables, où la deuxième branche est placée entre la paire d'étuis séparables de manière à pivoter autour de l'axe de pivotement.

4. Compas de mesure de pli cutané selon la revendication 1, **caractérisé en ce que** l'unité de sonde inclut :
les premières sondes entrant en contact avec une pluralité de premières échelles angulaires disposées de manière équidistantes au niveau d'un premier intervalle d'unité angulaire et
les deuxièmes sondes entrant en contact avec une pluralité de deuxièmes échelles angulaires disposées de manière équidistantes au niveau d'un deuxième intervalle d'unité angulaire, où la première unité angulaire est égale à un multiple de la deuxième unité angulaire par un nombre de la pluralité de deuxièmes échelles angulaires.

5. Compas de mesure de pli cutané selon la revendication 1, comprenant en outre un affichage prévu sur la première branche ou la deuxième branche pour l'affichage de la distance linéaire entre les extrémités de la première branche et de la deuxième branche calculée par le contrôleur.

6. Compas de mesure de pli cutané selon la revendication 1, comprenant en outre un module de communication prévu dans la première branche ou la deuxième branche, où le module de communication transmet, à un dispositif externe, les données de distance linéaire entre les extrémités de la première branche et de la deuxième branche calculées par le contrôleur ou l'information de composition du corps calculée automatiquement à partir des données de distance linéaire.

7. Compas de mesure de pli cutané selon la revendication 6, **caractérisé en ce que** le dispositif externe affiche les données de distance linéaire ou l'information de composition du corps calculée automatiquement à partir des données de distance linéaire.

8. Compas de mesure de pli cutané selon la revendication 1, comprenant en outre une batterie pour fournir de l'électricité à l'unité d'échelle, à l'unité de sonde, au détecteur de mesure de la pression et au contrôleur.
